# EUROPÄISCHE PATENTSCHRIFT

(11) **EP 2 958 546 B1**
(45) Veröffentlichungstag und Bekanntmachung des Hinweises auf die Patenterteilung: **22.11.2023**
(21) Anmeldenummer: 14706020.6
(22) Anmeldetag: 20.02.2014
(51) Int. Cl.: A61K 8/49, A61Q 17/04

(54) **MEDIKAMENT ZUT BEHANDLUNG DER DURCH UV-STRAHLEN HERVORGERUFENEN HAUTALTERUNG**
MEDICAMENT FOR TREATING THE SKIN AGEING CAUSED BY UV RADIATION
MÉDICAMENT POUR LE TRAITEMENT DU VIEILLISSEMENT DE LA PEAU INDUIT PAR LES RAYONS UV

(30) Priorität: 21.02.2013 EP 13156181
(43) Veröffentlichungstag der Anmeldung: 30.12.2015
(73) Patentinhaber: Symrise AG, 37603 Holzminden (DE)
(72) Erfinder: VIELHABER, Gabriele, F-75008 Paris (FR); LE MAIRE, Marielle, F-92100 Boulogne Billancourt (FR)
(74) Vertreter: Fabry, Bernd
(86) Internationale Anmeldenummer: PCT/EP2014/053365
(87) Internationale Veröffentlichungsnummer: WO 2014/128228

(56) Entgegenhaltungen:
- EP-A1- 1 591 105
- EP-A1- 2 545 898
- EP-A2- 1 310 238
- EP-A2- 2 181 697
- WO-A2-2010/124817
- NEELAM MUIZZUDDIN ET AL: "Effect of antioxidants and free radical scavengers on protection of human skin against UVB, UVA and IR irradiation", SKIN RESEARCH AND TECHNOLOGY, MUNKSGAARD, COPENHAGEN, DK, Bd. 5, 28. Dezember 1998 (1998-12-28), Seiten 260-265, XP002593991, ISSN: 0909-752X
- "Formulierungen", IP.COM JOURNAL, IP.COM INC., WEST HENRIETTA, NY, US, 26. Februar 2013 (2013-02-26), XP013156138, ISSN: 1533-0001
- FISHER G J ET AL: "PATHOPHYSIOLOGY OF PREMATURE SKIN AGING INDUCED BY ULTRAVIOLET LIGHT", THE NEW ENGLAND JOURNAL OF MEDICINE, MASSACHUSETTS MEDICAL SOCIETY, US, vol. 337, no. 20, 13 November 1997 (1997-11-13), pages 1419-1428, XP000938925, ISSN: 0028-4793, DOI: 10.1056/NEJM199711133372003

## Beschreibung

### GEBIET DER ERFINDUNG

Die Erfindung befindet sich auf dem Grenzgebiet zwischen Pharmazie und Kosmetik und betrifft ein Medikament zur Inhibierung der durch UV-Strahlung induzierten MMP-1-Expression mit Hilfe einer synergistischen Wirkstoffmischung.

### TECHNOLOGISCHER HINTERGRUND

Es wird seit langer Zeit angestrebt, die Hautalterung anzuhalten oder zumindest zu verzögern. Die Wissenschaft konnte als Ursachen der Hautalterung verschiedenste Beschleuniger aufzeigen, beispielsweise Schadstoffe aus der Umwelt, freie Radikale, oxidativen Stress, Entzündungen, ultraviolette Strahlung (UV-Strahlung) oder Infrarotstrahlung (IR-Strahlung).

Es werden ferner verschiedene Mechanismen der Hautalterung diskutiert, wobei sehr häufig enzymatische Mechanismen genannt werden und dem Enzym "Matrix-Metallo-Proteinase 1" (MMP-1) eine besondere Bedeutung zugesprochen wird. Eine vermehrte Expression oder Aktivierung von MMP-1 bzw. eine Steigerung der MMP-1-Aktivität werden im Allgemeinen als Beschleuniger der Hautalterung angesehen.

UV-Strahlung wird als wesentlicher Beschleuniger der Hautalterung angesehen, wobei deren Wirkung über verschiedenste Mechanismen hervorgerufen wird, etwa durch Schädigung der Erbsubstanz, Proteindenaturierung oder durch Induktion der MMP-1-Expression. Es gibt bisher sehr gute Möglichkeiten, die durch UV-Strahlung hervorgerufene Hautalterung z.B. durch die Verwendung von UV-Lichtschutzfaktoren zu vermindern, jedoch besteht ein ständiges Bedürfnis, die Wirksamkeit solcher Stoffe entweder durch Entwicklung neuer Filter oder synergistische Wirkstoffkombinationen zu verbessern.

### STAND DER TECHNIK

In diesem Zusammenhang sei auf die WO 2010124817 A2 (KAO) hingewiesen. Die Schrift betrifft Zubereitungen mit einem Gehalt an Dipeptiden. In den Bespielen 14 bis 16 werden Haarbehandlungsmittel offenbart, die auch L-Carnosin, jedoch keine Lichtschutzfaktoren enthalten.

Gegenstand der DE 10300782 A1 (BEIERSDORF) sind multiple Emulsionen mit einem Gehalt an Silikonverbindungen, die optional auch Carnotin oder Carnosin enthalten können. Die Zubereitungen weisen keine weiteren Lichtschutzfaktoren auf.

WO 2007122822 A1 (SHISEIDO) betrifft kosmetische Sonnenschutzemulsionen. In den Beispielen 22 und 23 werden Formulierungen offenbart, die auch Carnosin zusammen mit weiteren Lichtschutzfaktoren wie z.B. 5 Gew.-% Octocrylen enthalten. Die Schrift enthält jedoch keinen Hinweis auf die Eigenschaft von Carnosin, die Expression von MMP-1 zu inhibieren.

Aus der EP 1591105 A1 (STADA) sind kosmetische und pharmazeutische Zubereitungen bekannt, die ein Antioxidants enthalten, das die Haut vor IR-Strahlung schützt. Zu den geeigneten Antioxidantien gehören auch Carnosin und Arnesin.

Gegenstand der EP 2545898 A1 (COTY) sind Zubereitungen, die die Haut ebenfalls vor Schädigung durch IR-Strahlen schützen und dabei Pflanzenextrakte, Vitamine, Rubinpulver, Mica und Titandioxid enthalten. In Abschnitt [0026] wird als weiterer geeigneter Hilfsstoff Carnosin genannt.

Die EP 1310238 A2 (BASF) betrifft kosmetische bzw. dermatologische Lichtschutzzubereitungen, die spezielle Monomere bzw. Polymere in Kombination mit UV-Lichtschutzfaktoren enthalten. Darüber hinaus können weiter Antioxidantien, wie z.B. Carnosin zugegen sein.

Der Aufsatz mit dem Titel "Pathophysiology of premature skin ageing induced by UV light" von Fisher et al. in NEW ENGLAND J MEDICINE 337(20) p1419-1428 (1997**)** befasst sich mit der Bereitstellung von Präparaten zur Behandlung der UV-induzierten Hautalterung, die durch eine erhöhte Expression von MMPs ausgelöst wird. Die Behandlung der Haut mit Tretionin (Vitamin-A-Säure) hemmt die MMP-Expression und wirkt so der Hautalterung entgegen.

### AUFGABE DER ERFINDUNG

Es war daher vornehmliche Aufgabe der vorliegenden Erfindung, die durch UV-Strahlung verursachte Hautalterung zu reduzieren oder zu verhindern, entsprechende synergistische Wirkstoffmischungen dafür anzugeben und spezielle Formulierungen zur zielgerichteten Applikation der Wirkstoffe bereitzustellen.

Eine weitere Teilaufgabe hat darin bestanden, solche Zubereitungen zu entwickeln, die nicht nur hoch effizient sind, sondern auch eine besonders gute dermatologische Verträglichkeit aufweisen und insbesondere nicht zur Hautrötung, Hautbleichung oder Hautbräunung führen, nicht irritierend sind, die Haut nicht austrocknen oder rissig machen, sowie keine feuchten, schuppigen, pudrigen oder klebrigen Filme darauf hinterlassen.

Die neuen Zubereitungen sollten neben einem zielgerichteten Transport der erfindungsgemäßen Wirkstoffe zum Wirkungsort unter anderem dafür sorgen, dass die erfindungsgemäßen Wirkstoffe den Wirkungsort nach dem Auftragen sehr schnell erreichen und diese langanhaltend an den Wirkungsort abgegeben werden.

### BESCHREIBUNG DER ERFINDUNG

Gegenstand der Erfindung ist ein Medikament enthaltend
(a) mindestens eine Verbindung der Formel (I) 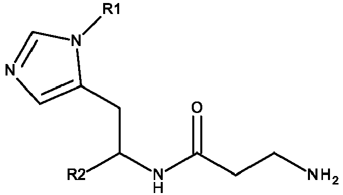 mit der Maßgabe, dass R1 für H oder CH₃ und R2 für H oder COOH steht, oder deren Salze, und
(b) mindestens einen UV-Lichtschutzfilter
zur Verwendung bei der Behandlung der durch UV-Strahlen hervorgerufenen Hautalterung.

Überraschenderweise wurde gefunden, dass Mischungen der Verbindungen der Formel (I), speziell Carnosin, in Kombination mit UV-Lichtschutzfaktoren, zu einer in synergistischer Weise verbesserten Inhibierung der MMP-1-Expression führen und damit das Leistungsvermögen konventioneller UV-Lichtschutzfaktoren in unerwarteter Weise verbessern.

Neben einer sehr hohen Effektivität der Wirkstoffe weisen diese eine sehr gute Verträglichkeit auf, führen nicht zur Hautrötung, Hautbleichung oder Hautbräunung, sind nicht irritierend, trocknen die Haut nicht aus, bilden keine feuchten, schuppigen, pudrigen oder klebrigen Filme darauf und machen die Haut nicht rissig. Die Stoffe zeichnen sich darüber hinaus dadurch aus, dass sie bei topischem Aufbringen langanhaltend am Wirkungsort verbleiben und so einen verbesserten UV-Schutz über einen zufriedenstellend langen Zeitraum garantieren.

### Wirkstoffe

Bei den Wirkstoffen, die die Formel (I) ausmachen, handelt es sich um grundsätzlich bekannte Verbindungen, die nach den üblichen Verfahren der organischen Chemie zugänglich sind. Vorzugsweise handelt es sich dabei um Gruppe von Stoffen, die gebildet wird von Carnosin, L-Carnosin, D-Carnosin, D/L-Carnosin, Carnicin, Carnicin*HCl, Anserin, D-Anserin, L-Anserin sowie L-Anserin*HNO₃ und deren Mischungen.

Unter Salzen der Verbindungen der Formel (I) werden erfindungsgemäß bevorzugt Salze der Verbindungen der Formel (I) mit mineralischen Säuren verstanden und besonders bevorzugt Salze der Formel (II): 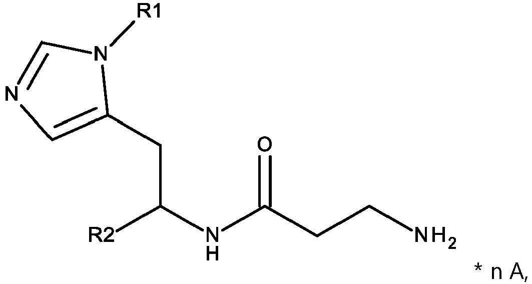 wobei n für 1,2 oder 3 und A für HCl oder HNO₃ steht und R1 für H oder CH3 sowie R2 für H oder COOH.

In besonderen Ausführungsformen der Erfindung finden die Verbindungen der Formel (I) oder deren Salze Verwendung zur Inhibierung der durch UV-Strahlung, speziell UV-A-Strahlung induzierten
- Synthese der MMP-1 codierenden mRNA oder
- Translation der MMP-1 aus der mRNA oder
- MMP-1 Aktivierung,
wobei die Inhibierung bevorzugt in oder an den Fibroblasten der Dermis (dermale Fibroblasten) erfolgt.

### UV-Lichtschutzfilter

Mischungen von Verbindungen der Formel (I) oder deren Salzen mit UV-Lichtschutzfaktoren führen zu einer synergistischen Verstärkung des Schutzes von Haut und Haaren gegen die schädliche Einwirkung von Sonnenlicht. Bei den UV-Lichtschutzfaktoren kann es sich dabei um UV-A-Filter, UV-B-Filter, Pigmente oder deren Gemische handeln, die im Folgenden weiter erläutert sind.

### UV-A- und UV-B-Lichtschutzfilter

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form langwelliger Strahlung, z.B. Wärme wieder abzugeben. Üblicherweise sind die UV-Lichtschutzfaktoren in Mengen von 0,1 bis 5 und vorzugsweise 0,2 bis 1 Gew.-% zugegen. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher beschrieben;
- 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)-benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-iso-propylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4methoxybenzophenon;
- Ester der Benzalmalonsäure, vorzugsweise 4-Methoxybenzmalonsäuredi-2-ethyl-hexylester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb^{®} HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- 1H-Benzimidazole-4,6-Disulfonic Acid, 2,2'-(1,4-Phenylene)Bis-, Disodium Salt (Neo Heliopan^{®} AP)
- Sulfonsäurederivate von Benzophenonen, vorzugsweise 2-Hydroxy-4-methoxybenzo-phenon-5-sulfonsäure und ihre Salze;
- Sulfonsäurederivate des 3-Benzylidencamphers, wie z.B. 4-(2-Oxo-3-bornylidenme-thyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxy-dibenzoylmethan (Parsol^{®} 1789), 2-(4-Diethylamino-2-hydroxybenzoyl)-benzoic acid hexylester (Uvinul^{®} A Plus), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans, z.B. 4-tert.-Butyl-4'-methoxydibenzoylmethan (Parsol^{®} 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäure-isoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Eine bevorzugte Ausführungsform besteht darin, dass im Fall, dass die Verbindung (I) Carnosin und der Lichtschutzfaktor Octocrylen ist, letzteres vorzugsweise nur in Mengen von unter 5 Gew.-% - bezogen auf die Zusammensetzung - vorhanden sein darf. Wird Benzophenon-3 verwendet, sollte die Zubereitung vorzugsweise frei von Chinonen sein.

### Lichtschutzpigmente

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex^{®} T2000, Eusolex^{®} T, Eusolex^{®} T-ECO, Eusolex^{®} T-S, Eusolex^{®} T-Aqua, Eusolex^{®} T-45D (alle Merck), Uvinul TiO₂ (BASF). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid wie z.B. Z-COTE^{®} oder Z-COTE HP1^{®} verwendet.

### Träger

Sowohl die Medikamente als auch die im Anschluss beschriebenen kosmetischen Zubereitungen können als Komponente (c) Träger bzw. Lösungsmittel enthalten, die ausgewählt sind aus der Gruppe, die gebildet wird von Wasser, Alkoholen, Estern, Butylenglycol, Dipropylenglycol, Ethanol, Ethoxydiglycol, Ethylacetat, Glycerin, Propanol, Isopropanol, Macrogole, Propylpropylenglycol(2)methylether, Propylpropyl-englycol(3)methylether, Propylencarbonat, Propylenglycol, Triethylenglycol, Isoparaffin, Amylacetat, Amylbenzoat, Benzylacetat, Butylacetat, Butylenglycol, Butyllactat, Butooctylbenzoat, Butooctylsalicylat, C10-C13 Alkane, C14-C17 Alkane, C11-C15 Cycloalkane, Caprylylbutyrat, Isoparaffine, Diacetin, Triacetin Dicaprylylether, Dicaprylylmaleat, und deren Mischungen.

### MEDIKAMENTE

Die erfindungsgemäßen Medikamente zur Verwendung gemäß den Ansprüchen enthalten die Komponenten (a) und (b) vorzugsweise im Gewichtsverhältnis von etwa 1:99 bis etwa 99:1, insbesondere von etwa 5:95 bis etwa 20:80 und ganz besonders bevorzugt von etwa 10:90 bis etwa 15:85. Die Synergie ist am stärksten ausgeprägt, wenn die beiden Komponenten etwa im Gewichtsverhältnis 1:5 eingesetzt werden.

Ein bevorzugtes Medikament zur Verwendung gemäß den Ansprüchen weist die folgende Zusammensetzung auf:
(a) etwa 0.05 bis etwa 19,5 Gew.-%, vorzugsweise etwa 1 bis etwa 15 Gew.-% und insbesondere etwa 2 bis etwa 8 Gew.-% einer Verbindung der Formel (I), vorzugsweise Carnosin,
(b) etwa 0.05 bis etwa 19,5 Gew.-%, vorzugsweise etwa 1 bis etwa 15 Gew.-% und insbesondere etwa 2 bis etwa 8 Gew.-% mindestens eines UV-Lichtschutzfaktors und
(c) ad 100 Gew.-% Träger.

### KOSMETISCHE ZUBEREITUNGEN

Die Mischungen enthaltend
(a) mindestens eine Verbindung der Formel (I) 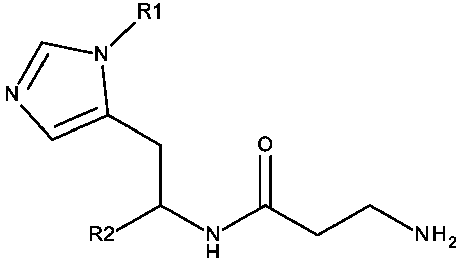 mit der Maßgabe, dass R1 für H oder CH₃ und R2 für H oder COOH steht, oder deren Salze, und
(b) mindestens einen UV-Lichtschutzfaktor sowie gegebenenfalls
(c1) Träger,
(c2) Ölkörper und/oder
(c3) Emulgatoren
können Bestandteile kosmetischer Zubereitungen sein. Diese liegen vorzugsweise als Cremes, Lotionen, Gele, Pasten oder Kapseln vor und stellen insbesondere Hautpflegemittel, Sonnenschutzmittel oder Haarpflegemittel handelt.

Weiterhin bevorzugt ist, dass die die Komponenten (a+b) bezogen auf die gesamte Zusammensetzung in Mengen von 0,1 bis 5 Gew.-% vorhanden sind. Dabei gelten die gleichen bevorzugten Gewichtsverhältnisse, wie sie schon oben beschrieben worden sind.

Die kosmetischen Mittel können weitere typische Hilfs- und Zusatzstoffe enthalten, wie beispielsweise milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, Feuchthaltemittel, biogene Wirkstoffe, Antioxidantien, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv^{®} TN), lineare oder verzweigte, symmetrische oder unsymmetrische Dialkylether mit 6 bis 22 Kohlenstoffatomen pro Alkylgruppe, wie z.B. Dicaprylyl Ether (Cetiol^{®} OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich oder Cosmedia^{®} SP von Cognis;
- Polyalkylenglycole sowie
- Glycerincarbonat.

Im Folgenden werden besonders geeignete Emulgatoren näher erläutert:
**Alkoxylate.** Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

**Alkyl- und/oder Alkenyloligoglykosid.** Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

**Partialglyceride.** Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

**Sorbitanester.** Als Sorbitanester kommen Sorbitanmonoisostearat, Sorbitansesquiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

**Polyglycerinester.** Typische Beispiele für geeignete Polyglycerinester sind Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Bellina^{®}, Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) und Polyglyceryl Polyricinoleate (Admul^{®} WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

**Anionische Emulgatoren.** Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

**Amphotere und kationische Emulgatoren.** Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine-COOH- oder -SOsH-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im Wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Kühlstoffe

Kühlstoffe sind Verbindungen, die auf der Haut ein Gefühlt der Kälte erzeugen. In der Regel handelt es sich dabei um Mentholverbindungen, die - neben dem Grundkörper Menthol selber - beispielsweise ausgewählt aus der Gruppe, die gebildet wird von Menthol Methyl Ether, Menthone Glyceryl Acetal (FEMA GRAS¹ 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.
¹ FEMA steht für "Flavor and Extracts Manufacturers Association" und GRAS ist definiert als "Generally Regarded As Safe". Eine FEMA GRAS Bezeichnung bedeutet, dass die so gekennzeichnete Substanz nach Standardmethode getestet und für toxikologisch unbedenklich erachtet wird.

Ein erster wichtiger Vertreter dieser Stoffe stellt das Monomenthyl Succinate (FEMA GRAS 3810) dar. Sowohl das Succinat als auch das analoge Monomenthyl Glutarate (FEMA GRAS 4006) stellen wichtige Vertreter von Monomenthylestern auf Basis von Di- und Polycarbonsäuren dar:

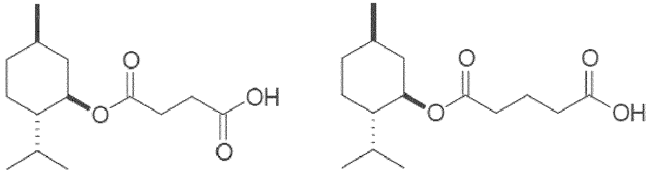

Beispiele für Anwendungen dieser Stoffe finden sich beispielsweise in den Druckschriften WO 2003 043431 (Unilever) oder EP 1332772 A1 (IFF).

Die nächste wichtige Gruppe von bevorzugten Mentholverbindungen umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat^{®} MGC), Menthol Propylenglycol Carbonate (FEMA GRAS 3784 = Frescolat^{®} MPC), Menthol 2-Methyl-1,2-propandiol Carbonate (FEMA GRAS 3849) oder den entsprechenden Zuckerderivaten. Ebenfalls bevorzugt sind die Mentholverbindungen Menthyl Lactate (FEMA GRAS 3748 = Frescolat^{®} ML) und insbesondere das Menthone Glyceryl Acetal (FEMA GRAS 3807) bzw. Menthone Glyceryl Ketal (FEMA GRAS 3808), das unter der Bezeichnung Frescolat^{®} MGA vermarktet wird. Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonatw erwiesen, die die Anmelderin unter den Bezeichnungen Frescolat^{®} MGA, Frescolat^{®} ML, Frecolat^{®} MGC und Frescolat^{®} MPC vertreibt.

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die bevorzugten Spezies WS-3, WS-4, WS-5, WS-12, WS-14 und WS-30.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole^{®} und Pemulen-Typen von Goodrich; Synthalene^{®} von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone^{®} Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel und Stabilisatoren

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400^{®} von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat^{®} (BASF), Kondensationsprodukte von Polyglycolen und Aminen, quaternierte Kollagenpolypeptide, wie beispielsweise Lauryldimonium Hydroxypropyl Hydrolyzed Collagen (La-mequat^{®}L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine^{®}/Sandoz), Copolymere der Acrylsäure mit Dimethyl-diallylammoniumchlorid (Merquat^{®} 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar^{®} CBS, Jaguar^{®} C-17, Jaguar^{®} C-16 der Firma Celanese, quaternierte Ammoniumsalz-Polymere, wie z.B. Mirapol^{®} A-15, Mirapol^{®} AD-1, Mirapol^{®} AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäurean-hydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmeth-acrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### Feuchthaltemittel

Feuchthaltemittel dienen zur weiteren Optimierung der sensorischen Eigenschaften der Zusammensetzung sowie zur Feuchtigkeitsregulierung der Haut. Gleichzeitig wird die Kältestabilität der Zubereitungen, insbesondere im Falle von Emulsionen, erhöht. Die Feuchthaltemittel sind üblicherweise in einer Menge von 0,1 bis 15 Gew.-%, vorzugsweise 1 bis 10 Gew.-%, und insbesondere 5 bis 10 Gew.-% enthalten.

Geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Bevorzugt geeignet als Feuchthaltemittel sind Glycerin, Diglycerin, Triglycerin und Butylenglycol.

### Biogene Wirkstoffe und Antioxidantien

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Filmbildner, Antischuppenwirkstoffe und Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival^{®} (Climbazole), Ketoconazol^{®}, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon^{®} UD (Protein-Undecylensäurekondensat), Zinkpyrithion, Aluminiumpyrithion und Magnesiumpyrithion / Dipyrithion-Magnesiumsulfat in Frage.

Als Quellmittel für wässrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in Cosm.Toil. 108. 95 (1993**)** entnommen werden.

### Insektenreepellentien

Als Insekten-Repellentien kommen N,N-Diethyl-m-toluamid, 1,2-Pentandiol oder Ethyl Butylacetylaminopropionate in Frage. Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschilderten Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine^{®} bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Bu-tylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden, wie sie beispielsweise in der Publikation **"**Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (CI.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### BEISPIELE

### Beispiele 1 bis 5. Vergleichsbeispiele V1 bis V4

### Inhibierung der MMP-1 Synthese

Untersucht wurde die Fähigkeit der Testsubstanzen den toxischen Einfluss von UVA-Strahlen zu mindern. Als in-vitro System diente eine Kultur dermaler Fibroblasten, bestimmt wurde die Freisetzung von MMP-1 aus diesen Fibroblasten unter dem Einfluss der UV-Strahlung. Zur Versuchsdurchführung wurde eine Fibroblastenkultur mit fötalem Kalbsserum angesetzt und 2 Tage später mit den Testsubstanzen geimpft. Nach einer Inkubation von 36 h bei 37 °C und einem CO₂-Level von 5 Vol.-% wurde das Nährmedium durch eine Elektrolytlösung ersetzt und die Fibroblasten mit einer definierten UVB-Strahlungsmenge geschädigt (50 mJ/cm²). Die Bestimmung der MMP erfolgte mit einem Kit, das unter der Bezeichnung RPN2610 von der Firma Amersham im Handel erhältlich ist. Die Ergebnisse sind in **Tabelle 1** zusammengefasst. Angegeben ist die Menge MMP in ng/ml aus einer Testreihe mit Dreifachbestimmung.

**Tabelle 1**

| **Bsp.** | **Testprodukte** | **Konz. % w/v** | **MMP** | |
|---|---|---|---|---|
| | | | **ohne UVA** | **mit UVA** |
| | Kontrolle | - | 49±9 | 199±25 |
| V1 | L-Carnosin | 0,001 | 22±5 | 101±17 |
| V2 | Carnicin*HCl | 0,001 | 24±7 | 118±21 |
| V3 | Uvinul A | 0,001 | 17±3 | 99±12 |
| V4 | Octocrinyl | 0,001 | 17±7 | 100±20 |
| 1 | L-Carnosin + Uvinul A (1:1) | 0,001 | 12±4 | 88±12 |
| 2 | L-Carnosin + Octocrinyl (1:1) | 0,001 | 14±3 | 92±15 |
| 3 | L-Carnosin + Uvasorb HEB (1:5) | 0,001 | 13±1 | 91±9 |
| 4 | L-Carnosin + NeoHeliopan (1:5) | 0,001 | 14±5 | 92±7 |
| 5 | L-Carnosin + Titandioxid (1:10) | 0,001 | 16±7 | 98±11 |

| | | | | |
|---|---|---|---|---|
| MMP-Inhibierung (Angabe in %-rel.) | | | | |

Die Ergebnisse zeigen, dass die Testsubstanzen die Freisetzung von MMP bei UVA-Bestrahlung nachhaltig und gegenüber den Einzelstoffen synergistisch vermindern

### ANWENDUNGSBEISPIELE

Die nachfolgenden Beispiele geben Formulierungen für unterschiedliche Sonnenschutzprodukte wieder, die die Mischungen der Komponenten (a) und (b) enthalten. Alle Mengenangaben verstehen sich als Gew.-%

### Kosmetisches Sonnenschutzmittel

| **Komponente** | **Menge** |
|---|---|
| Ethylhexyl cinnamic acid | 7.50 |
| Benzophenon-3 | 2.00 |
| Polyglyceryl dimer soyate | 0.80 |
| Sorbitane stearate | 1.00 |
| Tocopheryl acetate | 0.50 |
| Glyceryl stearate. PEG-100 Stearate | 3.00 |
| PEG-40. hydrogenated castor oil | 1.00 |
| Titanium dioxide. aluminum oxide hydrate. Dimethicon/Methicon Copolymer | 3.00 |
| *Butyrospermum parkii* (Shea Butter) | 1.00 |
| C₁₂₋₁₅ alkyl benzoate | 6.50 |
| Butylene glycol | 5.00 |
| Xanthan gum | 0.30 |
| Disodium EDTA | 0.10 |
| Allantoin | 0.10 |
| Polyacryl amide. C₁₃₋₁₄ isoparaffin. Laureth-7 | 1.00 |
| Pentylene glycol | 5.00 |
| 4-t Butylcyclohexanol | 1.00 |
| Carnosin | 0.30 |
| Preservatives (Methyl-. Butyl-. Ethyl-. Propylparaben. Phenoxyethanol) | 0.30 |
| Substance of formula (I) | 0.60 |
| Aqua dem. | Ad 100 |

### Sonnenschutzspray

| **Komponente** | **INCI** | **Menge** |
|---|---|---|
| Water, demineralized | Water (aqua) | 69.50 |
| Glycerol | Glycerol | 4.00 |
| 1.3 butylene glycol | Butylene glycol | 5.00 |
| D-Panthenol | Panthenol | 0.50 |
| Lara Care A-200 | Galactoarabinan | 0.25 |
| Baysilone oil M 10 | Dimethicone | 1.00 |
| Edeta BD | Disodium EDTA | 0.10 |
| Copherol 1250 | Tocopheryl acetate | 0.50 |
| Cetiol OE | Dicaprylyl ether | 3.00 |
| Neo Heliopan^{®} HMS | Homosalate | 5.00 |
| Neo Heliopan^{®} AV | Ethylhexyl methoxycinnamate | 6.00 |
| Neo Heliopan^{®} 357 | Butyl methoxydibenzoylmethane | 1.00 |
| Corapan TQ | Diethylhexylnaphthalate | 2.00 |
| Alpha Bisabolol | Bisabolol | 0.10 |
| Pemulen TR-2 | Acrylates/C10-30 alkyl acrylate crosspolymer | 0.25 |
| NaOH. 10% | Sodium hydroxide | 0.60 |
| Perfume oil | Fragrance | 0.20 |
| Phenoxyethanol | Phenoxyethanol | 0.40 |
| Solbrol M | Methylparaben | 0.10 |
| Solbrol P | Propylparaben | 0.10 |
| Carnosin | Carnosin | 0.50 |

### Sonnenschutzspray O/W SPF 15-20

| **Komponente** | **INCI** | **Menge** |
|---|---|---|
| Dracorin^{®} GOC | Glyceryl Oleate Citrate. Caprylic/Capric Triglyceride | 2.00 |
| Corapan^{®} TQ | Diethylhexyl 2.6-Naphthalate | 3.00 |
| Neo Heliopan^{®} HMS | Homosalate | 7.00 |
| Neo Heliopan^{®} OS | Ethylhexyl Salicylate | 5.00 |
| Neo Heliopan^{®} 357 | Butyl Methoxydibenzoylmethane | 3.00 |
| Isoadipate | Diisopropyl Adipate | 6.00 |
| Baysilone^{®} Oil M10 | Dimethicone | 1.00 |
| Edeta^{®} BD | Disodium EDTA | 0.10 |
| Vitamin E Acetate | Tocopheryl Acetate | 0.50 |
| Dragosantol^{®} 100 | Bisabolol | 0.10 |
| Pemulen^{®} TR-2 | Acrylates/C10-30 Alkyl Acrylate Crosspolymer | 0.25 |
| Water | Water (Aqua) | Ad 100 |
| Glycerol 99.5 P. | Glycerol | 4.00 |
| Butylene Glycol | Butylene Glycol | 5.00 |
| Neo Heliopan^{®} Hydro (103089). used as 25% aq. solution neutralized with Biotive^{®} L-Arginine | Phenylbenzimidazole Sulfonic Acid | 8.00 |
| Biotive^{®} L-Arginine | Arginine | 0.55 |
| Perfume oil | Fragrance | 0.40 |
| Sobrol M | Methylparaben | 0.30 |
| Carnosin | Carnosin | 0.60 |

### Sonnenschutzsoftcreme W/O SPF 40

| **Komponente** | **INCI** | **Menge** |
|---|---|---|
| Dehymuls PGPH | Polyglyceryl-2 dipolyhydroxystearate | 5.00 |
| Copherol 1250 | Tocopheryl acetate | 0.50 |
| Permulgin 3220 | Ozocerite | 0.50 |
| Zinc stearate | Zinc stearate | 0.50 |
| Tegosoft TN | C12-15 Alkyl benzoate | 10.00 |
| Neo Heliopan^{®} E1000 | Isoamyl-p-methoxycinnamate | 2.00 |
| Neo Heliopan^{®} 303 | Octocrylene | 5.00 |
| Neo Heliopan^{®} MBC | 4-Methylbenzylidene camphor | 3.00 |
| Zinc oxide. neutral | Zinc oxide | 5.00 |
| Water. distilled | Water (aqua) | Add 100 |
| EDETA BD | Disodium EDTA | 0.10 |
| Glycerol | Glycerol | 4.00 |
| Magnesium sulfate | Magnesium sulfate | 0.50 |
| Perfume oil P1. P2. P3 or P4 | Parfum | 0.30 |
| Symdiol^{®} 68 | 1.2-Hexanediol. Caprylylglycol | 0.30 |
| Carnosin | Carnosin | 0.80 |

### Sonnenschutzlotion W/O

| **Komponente** | **INCI** | **Menge** |
|---|---|---|
| Dehymuls PGPH | Polyglyceryl-2 dipolyhydroxystearate | 3.00 |
| Beeswax 8100 | Beeswax | 1.00 |
| Monomuls 90-0-18 | Glyceryl oleate | 1.00 |
| Zinc stearate | Zinc stearate | 1.00 |
| Cetiol SN | Cetearyl isononanoate | 5.00 |
| Cetiol OE | Dicaprylyl ether | 5.00 |
| Tegosoft TN | C12-15 alkyl benzoate | 4.00 |
| Vitamin E | Tocopherol | 0.50 |
| Neo Heliopan^{®} OS | Ethylhexyl salicylate | 5.00 |
| Neo Heliopan^{®} AV | Ethylhexyl methoxycinnamate | 7.50 |
| Uvinul^{®} T150 | Ethylhexyl triazone | 1.50 |
| Water. distilled | Water (Aqua) | ad 100 |
| Trilon BD | Disodium EDTA | 0.10 |
| Glycerol | Glycerol | 5.00 |
| Neo Heliopan^{®} AP 10% solution. neutralized with NaOH | Disodium phenyl dibenzimidazole tetrasulfonate | 15.00 |
| Perfume oil | Parfum | 0.25 |
| Alpha bisabolol | Bisabolol | 0.10 |
| SymOcide^{®} PT | Phenoxyethanol. Tropolone | 0.25 |
| Carnosin | Carnosin | 0.25 |

### Aftersun-Gel

| **Komponente** | **INCI** | **Menge** |
|---|---|---|
| SymSol^{®} PF-3 | Water (Aqua). Pentylene Glycol. Sodium Lauryl Sulfoacetate. SodiumOleoyl Sarcosinate. Sodium Chloride. Disodium Sulfoacetate. SodiumOleate. Sodium Sulfate | 3.00 |
| Glycerol 99.5 P. | Glycerol | 5.00 |
| SymHelios^{®} 1031 | Benzylidene Dimethoxydimethylin danone | 0.10 |
| Water | Water (Aqua) | Ad 100 |
| Pemulen^{®} TR-2 | Acrylates/C10-30 Alkyl Acrylate Crosspolymer | 1.00 |
| D-Panthenol 75 W | Panthenol | 0.50 |
| SymFinity^{®} 1298 | Echinacea Purpurea Extract | 0.10 |
| Extrapone^{®} Pearl GW | Water (Aqua). Glycerol. Hydrolyzed Pearl. Xanthan Gum | 1.00 |
| Sodium Hydroxide 10% solution | Sodium Hydroxide | 2.50 |
| Ethanol 96 % | Alcohol Denat. | 15.00 |
| Perfume oil | Parfum | 0.20 |
| SymOcide^{®} PS | Phenoxyethanol. 1.2-Hexanediol. Decyleneglycol | 0.50 |
| Carnosin | Carnosin | 0.10 |

## Patentansprüche

1. Medikament enthaltend
(a) mindestens eine Verbindung der Formel (I) 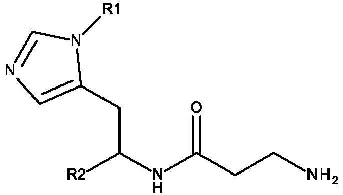 mit der Maßgabe, dass R1 für H oder CH₃ und R2 für H oder COOH steht, oder deren Salze, und
(b) mindestens einen UV-Lichtschutzfilter
zur Verwendung bei der Behandlung der durch UV-Strahlen hervorgerufenen Hautalterung.

2. Medikamente zur Verwendung nach Anspruch 1, **dadurch gekennzeichnet, dass** die Verbindung der Formel (I) ausgewählt ist aus der Gruppe die gebildet wird von Carnosin, L-Carnosin, D-Carnosin, D/L-Carnosin, Carnicin, Carnicin*HCl, Anserin, D-Anserin, L-Anserin sowie L-Anserin*HNO₃ und deren Mischungen.

3. Medikament zur Verwendung nach den Ansprüchen 1 und/oder 2, **dadurch gekennzeichnet, dass** die UV-Lichtschutzfilter ausgewählt sind aus der Gruppe, die gebildet wird von UV-A-Filtern, UV-B-Filtern und Lichtschutzpigmenten.

4. Medikament zur Verwendung nach Anspruch 3, **dadurch gekennzeichnet, dass** die UV-A-Filter ausgewählt sind aus der Gruppe, die gebildet wird von Benzoylmethan- und Enaminverbindungen sowie deren Gemischen.

5. Medikament zur Verwendung nach Anspruch 3, **dadurch gekennzeichnet, dass** die UV-B-Filter ausgewählt sind aus der Gruppe, die gebildet wird von 3-Benzylidencampher bzw. 3-Benzylidennorcampher und dessen Derivaten, 4-Aminobenzoesäurederivaten, Estern der Zimtsäure, Estern der Salicylsäure, Derivaten des Benzophenons, Estern der Benzalmalonsäure, Triazinderivaten, Propan-1,3-dionen, Ketotricyclo(5.2.1.0)decan-Derivaten, 2-Phenylbenzimidazol-5-sulfonsäure und deren Salzen; Sulfonsäurederivaten von Benzophenonen, Sulfonsäurederivaten des 3-Benzylidencamphers sowie deren Gemischen.

6. Medikament zur Verwendung nach Anspruch 3, **dadurch gekennzeichnet, dass** die Lichtschutzpigmente ausgewählt sind aus der Gruppe, die gebildet wird von Zinkoxid, Titandioxid, Eisenoxid(en), Zirkoniumoxid, Siliziumdioxid, Manganoxid, Aluminiumoxid, Ceroxid, Silikaten, Bariumsulfat und Zinkstearat sowie deren Gemischen.

7. Medikament zur Verwendung nach mindestens einem der Ansprüche 1 bis 6, **dadurch gekennzeichnet, dass** als optionale Komponente (c) Träger enthalten sind.

8. Medikament zur Verwendung nach Anspruch 7, **dadurch gekennzeichnet, dass** die Träger ausgewählt sind aus der Gruppe, die gebildet wird von Wasser, Alkoholen, Estern, Butylenglycol, Dipropylenglycol, Ethanol, Ethoxydiglycol, Ethylacetat, Glycerin, Propanol, Isopropanol, Macrogole, Propylpropylenglycol(2)methylether, Propylpropylenglycol(3)-methylether, Propylencarbonat, Propylenglycol, Triethylenglycol, Isoparaffin, Amylacetat, Amylbenzoat, Benzylacetat, Butylacetat, Butylenglycol, Butyllactat, Butooctylbenzoat, Butooctylsalicylat, C10-C13 Alkane, C14-C17 Alkane, C11-C15 Cycloalkane, Caprylylbutyrat, Isoparaffine, Diacetin, Triacetin Dicaprylylether, Dicaprylylmaleat, und deren Mischungen.

9. Medikament nach mindestens einem der Ansprüche 1 bis 8, **dadurch gekennzeichnet, dass** die Komponenten (a) und (b) im Gewichtsverhältnis 5:95 bis 20:80 vorhanden sind.

## Claims

1. A medicament containing
(a) at least one compound of formula (I) 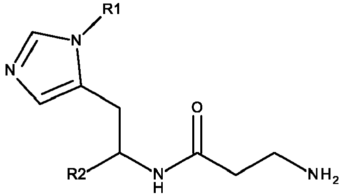 with the proviso that R1 is H or CH3 and R2 is H or COOH, or salts thereof, and
(b) at least one UV light protection filter
for use in the treatment of skin aging caused by UV radiation.

2. The medicaments for use according to claim 1, **characterized in that** the compound of the formula (I) is selected from the group formed by carnosine, L-carnosine, D-carnosine, D/L-carnosine, carnicine, carnicine*HCl, anserine, D-anserine, L-anserine and L-anserine*HNO3 and mixtures thereof.

3. The medicament for use according to claims 1 and/or 2, **characterized in that** the UV light protection filters are selected from the group formed by UV-A filters, UV-B filters and light protection pigments.

4. The medicament for use according to claim 3, **characterized in that** the UV-A filters are selected from the group formed by benzoylmethane and enamine compounds and mixtures thereof.

5. The medicament for use according to claim 3, **characterized in that** the UV-B filters are selected from the group formed by 3-benzylidenecamphor or 3-benzylidenenorcam-phor and derivatives thereof, 4-aminobenzoic acid derivatives, esters of cinnamic acid, esters of salicylic acid, derivatives of benzophenone, esters of benzalmalonic acid, triazine derivatives, propane-1,3-diones, ketotricyclo(5.2.1.0)decane derivatives, 2-phenylbenzimidazole-5-sulfonic acid and salts thereof; sulfonic acid derivatives of benzophenones, sulfonic acid derivatives of 3-benzylidenecamphor and mixtures thereof.

6. The medicament for use according to claim 3, **characterized in that** the light-protective pigments are selected from the group formed by zinc oxide, titanium dioxide, iron oxide(s), zirconium oxide, silicon dioxide, manganese oxide, aluminum oxide, cerium oxide, silicates, barium sulfate and zinc stearate, and mixtures thereof.

7. The medicament for use according to at least one of claims 1 to 6, **characterized in that** carriers are included as optional component (c).

8. The medicament for use according to claim 7, **characterized in that** the carriers are selected from the group formed by water, alcohols, esters, butylene glycol, dipropylene glycol, ethanol, ethoxydiglycol, ethyl acetate, glycerol, propanol, isopropanol, macrogols, propylene glycol (2) methyl ether, propylene glycol (3) methyl ether, propylene carbonate, propylene glycol, triethylene glycol, isoparaffin, amyl acetate, amyl benzoate, benzyl acetate, butyl acetate, butylene glycol, butyl lactate, butooctyl benzoate, butooctyl salicylate, C10-C13 alkanes, C14-C17 alkanes, C11-C15 cycloalkanes, caprylyl butyrate, isoparaffins, diacetin, triacetin dicaprylyl ether, dicaprylyl maleate, and mixtures thereof.

9. The medicament according to at least one of claims 1 to 8, **characterized in that** components (a) and (b) are present in a weight ratio of 5:95 to 20:80.

## Revendications

1. Un médicament contenant
(a) au moins un composé de formule (I) 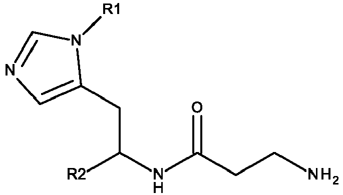 à condition que R1 représente H ou CH3 et R2 représente H ou COOH, ou leurs sels, et
(b) au moins un filtre de protection contre la lumière UV,
destiné à être utilisé dans le traitement du vieillissement cutané provoqué par les rayons UV.

2. Le médicament à utiliser selon la revendication 1, **caractérisé en ce que** le composé de formule (I) est choisi dans le groupe formé par la carnosine, la L-carnosine, la D-carnosine, la D/L-carnosine, la carnicine, la carnicine*HCl, l'ansérine, la D-ansérine, la L-ansérine ainsi que la L-ansérine*HNO3 et leurs mélanges.

3. Le médicament à utiliser selon les revendications 1 et/ou 2, **caractérisé en ce que** les filtres de protection contre la lumière UV sont choisis dans le groupe formé par les filtres UV-A, les filtres UV-B et les pigments de protection contre la lumière.

4. Le médicament destiné à être utilisé selon la revendication 3, **caractérisé en ce que** les filtres UV-A sont choisis dans le groupe formé par les composés de benzoylméthane et d'énamine ainsi que leurs mélanges.

5. Le médicament pour l'utilisation selon la revendication 3, **caractérisé en ce que** les filtres UV-B sont choisis dans le groupe formé par le 3-benzylidène camphre ou le 3-benzylidène norbocamphre et ses dérivés, les dérivés de l'acide 4-aminobenzoïque, les esters de l'acide cinnamique, les esters de l'acide salicylique, les dérivés de la benzophénone, les esters de l'acide benzalmalonique, les dérivés de la triazine, les propane-1,3-diones, le cétotricyclo(5.2.1.0)décane, l'acide 2-phénylbenzimidazole-5-sulfonique et leurs sels ; les dérivés d'acide sulfonique de benzophénones, les dérivés d'acide sulfonique du 3-benzylidène camphre ainsi que leurs mélanges.

6. Le médicament pour l'utilisation selon la revendication 3, **caractérisé en ce que** les pigments de protection contre la lumière sont choisis dans le groupe formé par l'oxyde de zinc, le dioxyde de titane, le ou les oxydes de fer, l'oxyde de zirconium, le dioxyde de silicium, l'oxyde de manganèse, l'oxyde d'aluminium, l'oxyde de cérium, les silicates, le sulfate de baryum et le stéarate de zinc ainsi que leurs mélanges.

7. Le médicament destiné à être utilisé selon au moins l'une des revendications 1 à 6, **caractérisé en ce que** des supports sont contenus en tant que composant optionnel (c).

8. Le médicament pour utilisation selon la revendication 7, **caractérisé en ce que** les supports sont choisis dans le groupe formé par l'eau, les alcools, les esters, le butylèneglycol, le dipropylèneglycol, l'éthanol, l'éthoxydiglycol, l'acétate d'éthyle, le glycérol, le propanol, l'isopropanol, les macrogols, l'éther méthylique de propylpropylèneglycol (2), l'éther méthylique de propylpropylèneglycol (3), carbonate de propylène, propylène glycol, triéthylène glycol, isoparaffine, acétate d'amyle, benzoate d'amyle, acétate de benzyle, acétate de butyle, butylène glycol, lactate de butyle, benzoate de butooctyle, butooctyl-salicylate, alcanes en C10-C13, alcanes en C14-C17, cycloalcanes en C11-C15, butyrate de caprylyle, isoparaffines, diacétine, triacétine dicaprylyléther, maléate de dicaprylyle, et leurs mélanges.

9. Le médicament selon au moins l'une des revendications 1 à 8, **caractérisé en ce que** les composants (a) et (b) sont présents dans un rapport pondéral de 5:95 à 20:80.
